# EUROPEAN PATENT SPECIFICATION

(11) **EP 1 467 718 B1**
(45) Date of publication and mention of the grant of the patent: **23.11.2005**
(21) Application number: 03708305.2
(22) Date of filing: 22.01.2003
(51) Int. Cl.: A61K 9/48

(54) **METHOD OF TREATMENT OF A PATIENT REQUIRING ANALGESIA**
METHODE ZUR BEHANDLUNG EINES ANALGESIEBEDÜRFTIGEN PATIENTEN
METHODE DE TRAITEMENT D'UN PATIENT NECESSITANT UNE ANALGESIE

(30) Priority: 22.01.2002 GB 0201367
(43) Date of publication of application: 20.10.2004
(73) Proprietor: ML Laboratories Plc, London W1S 1HU (GB)
(72) Inventor: JACKSON, Karen, Deepcar, Sheffield S35 2ND (GB)
(74) Representative: Harrison Goddard Foote
(86) International application number: PCT/GB2003/000221
(87) International publication number: WO 2003/061632

(56) References cited:
- WO-A-99/18967
- GB-A- 1 564 039
- US-A- 5 153 191

## Description

This invention relates to a novel method of treatment and a novel pharmaceutical composition related thereto.

International Patent Application No. WO 99/18967 describes pharmaceutical compositions for treating chronic and neuropathic pain which comprises an analgesic amount of an opioid and an opioid potentiating amount of a CCK antagonist. WO '967 describes the use of both CCK-A (CCK-1) antagonists and CCK-B (CCK-2) antagonists, although it is described that, generally, CCK-B (CCK-2) antagonists are preferred. Moreover, page 2, lines 6 to 8 of WO '967 describes that CCK-A (CCK-1) antagonists may be suitable, but only at relatively higher dosages.

One specific CCK-A (CCK-1) antagonist which is mentioned in WO 99/18967 is devazepide (Devacade®), which is 3s-(-)-1,3-dihydro-3-(2-indolecarbonylamino)-1-methyl-5-phenyl-2H-1,4-benzodiazepin-2-one.

Devazepide is commonly administered alongside an opioid analgesic, e.g. such as morphine. However, in normal doses, the commonest side-effects of morphine and other opioid analgesics are nausea, vomiting, constipation, drowsiness, and confusion; tolerance generally develops with long-term use, but not to constipation which is the most common undesirable side effect of morphine treatment.

International Patent Application No. WO 99/18967 specifically describes a pharmaceutical formulation comprising a CCK antagonist, such as devazepide, an opioid and a biphasic carrier, comprising a glyceride derivative organic phase. This application suggests the possible use of a surfactant, especially when the formulation is in the form of an oil-in-water emulsion.

We have now surprisingly found that a method of treatment of a patient requiring analgesia which comprises administering a monophasic form of devazepide which may be prepared with a surfactant. The use of a surfactant is advantageous in that, *inter alia*, it improves the powder flow and/or separation properties of solid devazepide and also reduces or mitigates the undesirable side effects of opioid administration, e.g. constipation.

Thus, according to the invention we provide a composition for the treatment of a patient requiring analgesia which comprises the separate, simultaneous or sequential administration of a therapeutically effective amount of an opioid analgesic, devazepide and a pharmaceutically acceptable surfactant wherein the daily dosage of devazepide is up to 0.7 mg/kglday.

The composition of the invention especially provides a composition wherein the devazepide and the pharmaceutically acceptable surfactant are in a monophasic form, e.g. solid or liquid form. Preferably, the devazepide and the pharmaceutically acceptable surfactant are in a monophasic form, eg a liquid form or a solid dosage form. The phrase solid dosage form may mean, for example, in tablet form or, preferably in the form of a flowable powder in a capsule. We have found that the use of a surfactant in a solid dose devazepide composition as hereinbefore described has the advantage of mitigating constipation due to the concomitant administration of an opioid analgesic, whilst also improving the physical properties of devazepide n a solid dose formulation.

In an especially preferred embodiment of the invention the surfactant will be capable of improving powder flow of devazepide and may be known to be a therapeutically effective laxative and/or stool softener. Such laxatives and/or stool softeners may, preferentially be ionic surfactants, especially alkyl sulphosuccinates, alkyl sulphates or alkyl ammonium salts.

Thus, in a preferred embodiment of the invention the surfactant may be selected from the group, docusate sodium (dioctyl sodium sulphosuccinate), sodium dodecyl sulphate and tetradecyltrimethyl ammonium bromide.

In a further embodiment of the invention the surfactant may also possess antimicrobial and/or antiseptic properties. Thus, for example, when the surfactant is tetradecyltrimethylammonium bromide, it may, preferentially, be cetrimide (cetrimide is a mixture substantially comprising tetradecyltrimethyl ammonium bromide and small amounts of dodecyltrimethylammonium bromide and cetrimonium bromide).

In the most preferred embodiment of the invention the surfactant is docusate sodium.

The composition may preferentially comprise the use of a composition which comprises one or more fillers. Thus, such fillers may be selected from the group lactose, mannitol, talc, magnesium stearate, sodium chloride, potassium chloride, citric acid, spray-dried lactose, hydrolysed starches, directly compressible starch, microcrystalline cellulose, cellulosics, sorbitol, sucrose, sucrose-based materials, icodextrin, calcium sulphate, dibasic calcium phosphate and dextrose. A preferred filler is starch, e.g. corn starch.

When the invention comprises the use of a composition which includes a filler, the size of the devazepide and filler particles may be the same or different. However, in a preferred embodiment the sizes of the devazepide and filler particles will differ. Preferentially, the devazepide, surfactant and/or the filler may be of reduced particle size, e.g. by milling.

The devazepide, surfactant and filler may be present as an intimate mixture. However, in a preferred embodiment the filler particles may be coated with the surfactant, the coated filler and devazepide then being formed into an intimate mixture.

The invention wherein the compositions, comprising devazepide, a filler and a surfactant are especially advantageous in that, *inter alia*, the surfactant acts to hinder or prevent separation of the devazepide and the filler. Furthermore, in one embodiment of the invention the surfactant may also have desirable laxative and/or stool softening properties.

The amount of surfactant present in the composition used in the method of the invention may vary, depending upon, *inter alia*, the level of devazepide present, the level of concomitant opioid analgesic administered, etc. Generally, the ratio of devazepide:surfactant may be from 5:1 to 2 5:1 w /w, preferably from 10:1 to 15:1 w/w, most preferably 12.5:1 w/w.

When the composition used in the invention includes a filler, the composition may generally comprise devazepide and a surfactant, in the ratio as hereinbefore described, with the remainder of the composition being made up with a filler.

A preferred embodiment of the invention comprises a composition as hereinbefore described is filled into a capsule. Any conventionally known materials may be used for the capsule, however a preferred material is gelatin.

Thus, for example, in one embodiment of the invention the composition as hereinbefore described may be made up into a capsule formulation, e.g. with a fill weight of 150 mg ± 5% by weight or 300 mg ± 5% by weight. In the one preferred embodiment, the capsule formulation may comprise 1.25mg devazepide, 0.1 mg surfactant, e.g. d ocusate s odium, and 148.65 mg of a filler, e.g. corn starch. In a further preferred embodiment, the capsule formulation may comprise 2.5mg devazepide, 0.2 mg surfactant, e.g. docusate sodium, and 297.3 mg of a filler, e.g. corn starch.

According to a further aspect of the invention we provide the use of devazepide in the manufacture of a pharmaceutical composition comprising a therapeutically effective amount of devazepide and a pharmaceutically acceptable surfactant.

The use of the invention is preferentially the use in the manufacture of a pharmaceutical composition wherein the composition comprises any of the aspects of the methods hereinbefore described. The use as hereinbefore described preferentially comprises the use in the manufacture of a pharmaceutical composition in monophasic form.

By the term therapeutically effective amount of devazepide we generally mean an amount of devazepide effective in the enhancement of opioid analgesia.

In the compositions of the invention a variety of opioids may be used. Thus, the opioid may be selected from those which are effective analgesics and particularly those which need to be administered at relatively high or increasing doses. Examples include morphine, or a salt thereof such as the sulphate, chloride or hydrochloride, or the other 1,4-hydroxymorphinan opioid analgesics such as naloxone, meperidine, butorphanol or pentazocine, or morphine-6-glucuronide, codeine, dihydrocodeine, diamorphine, dextropropoxyphene, pethidine, fentanyl, alfentanil, alphaprodine, buprenorphine, dextromoramide, diphenoxylate, dipipanone, heroin (diacetylmorphine), hydrocodone (dihydrocodeinone), hydromorphone (dihydromorphinone), levorphanol, meptazinol, methadone, metopon (methyldihydromorphinone), nalbuphine, oxycodone (dihydrohydroxycodeinone), oxymorphone (dihydrohydroxymorphinone), phenadoxone, phenazocine, remifentanil, tramadol, or a salt of any of these. The opioid used in the method of the invention may comprise any combination of the aforementioned compounds. Naloxone is also included within the definition of an opioid. Especially preferred analgesics which may be mentioned are hydromorphone, oxycodone, morphine, e.g. morphine sulphate and fentanyl. In a preferred embodiment of the invention the analgesic is morphine or morphine sulphate. In a further preferred embodiment the opioid is fentanyl or a salt thereof.

In the compositions of the invention the devazepide and/or the opioid may be administered using any methods conventionally known *per se.* Thus, such methods would include, but shall not be limited to, administration intravenously, intra-arterially, orally, intrathecally, intranasally, intrarectally, intramuscularly/subcutaneously, by inhalation and by transdermal patch. When the devazepide and/or opioid is administered intravenously, it may, for example, be as an intravenous bolus or a continuous intravenous infusion. When the devazepide and/or the opioid is administered subcutaneously, it may for example be by subcutaneous infusion. Preferably, the opioid and/or devazepide are administered intravenously or orally. Oral administration is especially preferred. In a further preferred embodiment the opioid may be administered by a transdermal patch. When a transdermal patch is used, the preferred opioid is fentanyl or a salt thereof.

Thus, in the compositions of the invention the daily dosage of devazepide may vary depending upon, *inter alia*, the weight of the patient, the method of administration, etc. In patients that are suffering serious disorders, such as cancer patients, the weight of the patient may be very low and therefore the dosage of devazepide consequentially may be low. Preferably, the daily dosage of devazepide may be from 25 µg/kg/day to 0.7 mg/kg/day, more preferably from 50 µg/kg/day to 0.5 mg/kg/day. For oral administration the daily dosage of devazepide may be from 0.07 mg/kg/day to 0.7 mg/kg/day, preferably 0.07 mg/kg/day to 0.29 mg/kg/day. For intravenous administration the dosage of devazepide is preferably 50 µg/kg/day to 0.5 mg/kg/day.

Thus, the expected daily dose of surfactant, which may optionally have laxative and/or stool softening properties, may be up to 0.056 mg/kg/day. Thus, dependant upon the patient, the daily dosage of surfactant may be from 0.4mg to 1.6mg, preferably 0.8mg. Most preferably, the surfactant will be one which posses both laxative and stool softening properties.

In the compositions of the invention the dosage of the opioid analgesic administered may vary depending upon, *inter alia*, the nature of the opioid analgesic, the weight of the patient, the method of administration, etc. Thus, for example, the dosage of, e.g. an opioid, such as morphine, may be from 5 to 2000mg daily. A particular dosage which may be mentioned is from 10 to 240mg daily. A daily dosage of morphine may be from 5 to 100mg or occasionally up to 500mg.

According to a yet further aspect of the invention we provide a monophasic pharmaceutical composition comprising an amount of devazepide effective in the enhancement of opioid analgesia and a pharmaceutically acceptable surfactant.

Preferably, the devazepide and the pharmaceutically acceptable surfactant are in a solid dosage form. The phrase solid dosage form may mean, for example, in tablet form or, preferably in the form of a flowable powder in a capsule.

The composition of this aspect of the invention is preferentially a composition which comprises any of the aspects of the methods hereinbefore descried.

The devazepide used in the method and/or the composition of the invention is the S enantiomer, preferentially, the S enantiomer wherein the level of R enantiomer, which may be present as an impurity, is not greater than 1.5% w/w.

## Claims

1. The use of devazepide in the manufacture of a monophasic pharmaceutical composition comprising a therapeutically effective amount of devazepide and a pharmaceutically acceptable surfactant wherein the daily dosage of devazepide is up to 0.7 mg/kg/day **characterised in that** the surfactant is selected from the group alkyl sulphosuccinates, alkyl sulphates or alkyl ammonium salts.

2. The use according to claim 1 **characterised in that** the pharmaceutical composition is in solid dosage form.

3. A monophasic pharmaceutical composition comprising a therapeutically effective amount of devazepide and a pharmaceutically acceptable surfactant wherein the daily dosage of devazepide is up to 0.7 mg/kg/day **characterised in that** the surfactant is selected from the group alkyl sulphosuccinates, alkyl sulphates or alkyl ammonium salts.

4. A monophasic pharmaceutical composition according to claim 3 **characterised in that** the daily dosage of devazepide is from 25 µg/kg/day to 0.7 mg/kg/day.

5. A monophasic pharmaceutical composition according to claim 4 **characterised in that** the daily dosage of devazepide is from 50 µg/kg/day to 0.5 mg/kg/day.

6. A monophasic pharmaceutical composition according to claim 3 **characterised in that** the composition is in a liquid form.

7. A monophasic pharmaceutical composition according to claim 3 **characterised in that** the composition is in a solid dosage form.

8. A monophasic pharmaceutical composition according to claim 7 **characterised in that** the composition is in the form of a tablet.

9. A monophasic pharmaceutical composition according to claim 7 **characterised in that** the composition is in the form of a flowable powder in a capsule.

10. A monophasic pharmaceutical composition according to claim 3 **characterised in that** the composition is adapted for the separate, simultaneous or sequential administration with a therapeutically effective amount of an opioid analgesic.

11. A monophasic pharmaceutical composition according to claim 3 **characterised in that** the composition is adapted to be administered intravenously, intra-arterially, orally, intrathecally, intranasally, intrarectally, intramuscularly/subcutaneously, by inhalation or by transdermal patch.

12. A monophasic pharmaceutical composition according to claim 11 **characterised in that** the devazepide and/or the opioid is adapted to be administered intravenously.

13. A monophasic pharmaceutical composition according to claim 12 **characterised in that** the intravenous administration is by intravenous bolus or a continuous intravenous infusion.

14. A monophasic pharmaceutical composition according to claim 11 **characterised in that** the devazepide and/or the opioid is adapted to be administered subcutaneously.

15. A monophasic pharmaceutical composition according to claim 14 **characterised in that** the subcutaneous administration is as a subcutaneous infusion.

16. A monophasic pharmaceutical composition according to claim 11 **characterised in that** the devazepide and/or the opioid is adapted to be administered orally.

17. A monophasic pharmaceutical composition according to claim 11 **characterised in that** the devazepide is administered orally.

18. A monophasic pharmaceutical composition according to claim 12 **characterised in that** the opioid is administered intravenously and the devazepide is administered intravenously.

19. A monophasic pharmaceutical composition according to claim 16 **characterised in that** the opioid is administered orally and the devazepide is administered orally.

20. A monophasic pharmaceutical composition according to claim 11 **characterised in that** the opioid is administered by intravenous administration or oral administration.

21. A monophasic pharmaceutical composition according to claim 11 **characterised in that** the opioid is administered by transdermal patch.

22. A monophasic pharmaceutical composition according to claim 11 **characterised in that** for oral administration the daily dosage of devazepide is from 0.07 mg/kg/day to 0.7 mg/kg/day.

23. A monophasic pharmaceutical composition according to claim 22 **characterised in that** for oral administration the daily dosage of devazepide is from 0.07 mg/kg/day to 0.29 mg/kg/day.

24. A monophasic pharmaceutical composition according to claim 23 **characterised in that** for intravenous administration the dosage of devazepide is 50 µg/kg/day to 0.5 mg/kg/day.

25. A monophasic pharmaceutical composition according to claim 10 **characterised in that** the opioid is selected from the group morphine, or a salt thereof such as the sulphate, chloride or hydrochloride, or the other 1,4-hydroxymorphinan opioid analgesics such as naloxone, meperidine, butorphanol or pentazocine, or morphine-6-glucuronide, codeine, dihydrocodeine, diamorphine, dextropropoxyphene, pethidine, fentanyl, alfentanil, alphaprodine, buprenorphine, dextromoramide, diphenoxylate, dipipanone, heroin (diacetylmorphine), hydrocodone (dihydrocodeinone), hydromorphone (dihydromorphinone), levorphanol, meptazinol, methadone, metopon (methyldihydromorphinone), nalbuphine, oxycodone (dihydrohydroxycodeinone), oxymorphone (dihydrohydroxymorphinone), phenadoxone, phenazocine, remifentanil, tramadol, or a salt of any of these, or any combination of the aforementioned compounds.

26. A monophasic pharmaceutical composition according to claim 25 **characterised in that** the opioid is selected from the group hydromorphone, oxycodone, morphine and fentanyl, or a salt thereof.

27. A monophasic pharmaceutical composition according to claim 26 **characterised in that** the opioid is morphine or morphine sulphate.

28. A monophasic pharmaceutical composition according to claim 26 **characterised in that** the opioid is fentanyl, or a salt thereof.

29. A monophasic pharmaceutical composition according to claim 3 **characterised in that** the daily dose of surfactant is up to 0.056 mg/kg/day.

30. A monophasic pharmaceutical composition according to claim 3 **characterised in that** the daily dose of surfactant is from 0.4mg to 1.6mg per day.

31. A monophasic pharmaceutical composition according to claim 10 **characterised in that** the dosage of an opioid is from 5 to 2000mg daily.

32. A monophasic pharmaceutical composition according to claim 31 **characterised in that** the dosage of the opioid is from 10 to 240mg daily.

33. A monophasic pharmaceutical composition according to claim 32 **characterised in that** the daily dosage of the opioid is from 5 to 100mg daily.

34. A monophasic pharmaceutical composition according to claim 3 **characterised in that** the devazepide is substantially the S enantiomer.

35. A monophasic pharmaceutical composition according to claim 34 **characterised in that** the level of R enantiomer, which may be present as an impurity, is not greater than 1.5% w/w.

36. A monophasic pharmaceutical composition according to claim 3 **characterised in that** the surfactant is selected from the group, docusate sodium (dioctyl sodium sulphosuccinate), sodium dodecyl sulphate and tetradecyltrimethyl ammonium bromide.

37. A monophasic pharmaceutical composition according to claim 3 **characterised in that** the surfactant also possesses antimicrobial and/or antiseptic properties.

38. A monophasic pharmaceutical composition according to claim 37 **characterised in that** the surfactant is cetrimide.

39. A monophasic pharmaceutical composition according to claim 36 **characterised in that** the surfactant is docusate sodium.

40. A monophasic pharmaceutical composition according to claim 3 **characterised in that** the composition comprises one or more fillers.

41. A monophasic pharmaceutical composition according to claim 40 **characterised in that** the filler particles are coated with the surfactant, the coated filler and devazepide then being formed into an intimate mixture.

42. A monophasic pharmaceutical composition according to claim 40 **characterised in that** the filler is selected from the group lactose, mannitol, talc, magnesium stearate, sodium chloride, potassium chloride, citric acid, spray-dried lactose, hydrolysed starches, starch, microcrystalline cellulose, cellulosics, sorbitol, sucrose, sucrose-based materials, icodextrin, calcium sulphate, dibasic calcium phosphate and dextrose and mixtures thereof.

43. A monophasic pharmaceutical composition according to claim 42 **characterised in that** the filler is starch.

44. A monophasic pharmaceutical composition according to claim 43 **characterised in that** the starch is corn starch.

45. A monophasic pharmaceutical composition according to claim 40 **characterised in that** the size of the devazepide particles and the filler particles are different.

46. A monophasic pharmaceutical composition according to claim 3 **characterised in that** the ratio of devazepide: surfactant is from 5:1 to 25:1 w/w.

47. A monophasic pharmaceutical composition according to claim 40 **characterised in that** the composition comprises devazepide and a surfactant with the remainder of the composition being made up with a filler.

48. A monophasic pharmaceutical composition according to claim 47 **characterised in that** the composition comprises 1.25mg devazepide, 0.1 mg surfactant and 148.65 mg of a filler.

49. A monophasic pharmaceutical composition according to claim 48 **characterised in that** the composition comprises 1.25mg devazepide, 0.1 mg docusate sodium and 148.65 mg of corn starch.

50. A monophasic pharmaceutical composition according to claim 47 **characterised in that** the composition comprises is 2.5mg devazepide, 0.2 mg surfactant and 297.3mg of a filler.

51. A monophasic pharmaceutical composition according to claim 50 **characterised in that** the composition comprises 2.5mg devazepide, 0.2mg docusate sodium and 297.3mg corn starch.

52. A monophasic pharmaceutical composition according to claim 3 **characterised in that** the composition is filled into a capsule.

53. A monophasic pharmaceutical composition according to claim 52 **characterised in that** the capsule is a gelatin capsule.

## Patentansprüche

1. Verwendung von Devazepid bei der Herstellung einer monophasischen pharmazeutischen Zusammensetzung, die eine therapeutisch wirksame Menge Devazepid und einen pharmazeutisch verträglichen Oberflächenaktivstoff umfasst, worin die Tagesdosis von Devazepid bis zu 0,7 mg/kg/Tag beträgt, **dadurch gekennzeichnet, dass** der Oberflächenaktivstoff ausgewählt ist aus der Gruppe Alkylsulfosuccinate, Alkylsulfate oder Alkylammoniumsalze.

2. Verwendung gemäß Anspruch 1, **dadurch gekennzeichnet, dass** die pharmazeutische Zusammensetzung als feste Darreichungsform vorliegt.

3. Monophasische pharmazeutische Zusammensetzung, die eine therapeutisch wirksame Menge Devazepid und einen pharmazeutisch verträglichen Oberflächenaktivstoff umfasst, worin die Tagesdosis von Devazepid bis zu 0,7 mg/kg/Tag beträgt, **dadurch gekennzeichnet, dass** der Oberflächenaktivstoff ausgewählt ist aus der Gruppe Alkylsulfosuccinate, Alkylsulfate oder Alkylammoniumsalze.

4. Monophasische pharmazeutische Zusammensetzung gemäß Anspruch 3, **dadurch gekennzeichnet, dass** die Tagesdosis von Devazepid von 25 µg/kg/Tag bis 0,7 mg/kg/Tag beträgt.

5. Monophasische pharmazeutische Zusammensetzung gemäß Anspruch 4, **dadurch gekennzeichnet, dass** die Tagesdosis von Devazepid von 50 µg/kg/Tag bis 0,5 mg/kg/Tag beträgt.

6. Monophasische pharmazeutische Zusammensetzung gemäß Anspruch 3, **dadurch gekennzeichnet, dass** die Zusammensetzung in einer flüssigen Form vorliegt.

7. Monophasische pharmazeutische Zusammensetzung gemäß Anspruch 3, **dadurch gekennzeichnet, dass** die Zusammensetzung in einer festen Darreichungsform vorliegt.

8. Monophasische pharmazeutische Zusammensetzung gemäß Anspruch 7, **dadurch gekennzeichnet, dass** die Zusammensetzung in Form einer Tablette vorliegt.

9. Monophasische pharmazeutische Zusammensetzung gemäß Anspruch 7, **dadurch gekennzeichnet, dass** die Zusammensetzung in Form eines fließfähigen Pulvers in einer Kapsel vorliegt.

10. Monophasische pharmazeutische Zusammensetzung gemäß Anspruch 3, **dadurch gekennzeichnet, dass** die Zusammensetzung für eine separate, simultane oder sequentielle Verabreichung mit einer therapeutisch wirksamen Menge eines Opioid-Analgetikums angepasst ist.

11. Monophasische pharmazeutische Zusammensetzung gemäß Anspruch 3, **dadurch gekennzeichnet, dass** die Zusammensetzung angepasst ist, um intravenös, intraarteriell, oral, intrathekal, intranasal, intrarektal, intramuskulär/subkutan, mittels Inhalation oder mittels transdermalen Pflasters verabreicht zu werden.

12. Monophasische pharmazeutische Zusammensetzung gemäß Anspruch 11, **dadurch gekennzeichnet, dass** das Devazepid und/oder das Opioid angepasst ist beziehungsweise angepasst sind, um intravenös verabreicht zu werden.

13. Monophasische pharmazeutische Zusammensetzung gemäß Anspruch 12, **dadurch gekennzeichnet, dass** die intravenöse Verabreichung mittels intravenösem Bolus oder einer kontinuierlichen intravenösen Injektion erfolgt.

14. Monophasische pharmazeutische Zusammensetzung gemäß Anspruch 11, **dadurch gekennzeichnet, dass** das Devazepid und/oder das Opioid angepasst ist beziehungsweise sind, um subkutan verabreicht zu werden.

15. Monophasische pharmazeutische Zusammensetzung gemäß Anspruch 14, **dadurch gekennzeichnet, dass** die subkutane Verabreichung als subkutane Infusion erfolgt.

16. Monophasische pharmazeutische Zusammensetzung gemäß Anspruch 11, **dadurch gekennzeichnet, dass** das Devazepid und/oder das Opioid angepasst ist beziehungsweise angepasst sind, um oral verabreicht zu werden.

17. Monophasische pharmazeutische Zusammensetzung gemäß Anspruch 11, **dadurch gekennzeichnet, dass** das Devazepid oral verabreicht wird.

18. Monophasische pharmazeutische Zusammensetzung gemäß Anspruch 12, **dadurch gekennzeichnet, dass** das Opioid intravenös verabreicht wird und das Devazepid intravenös verabreicht wird.

19. Monophasische pharmazeutische Zusammensetzung gemäß Anspruch 16, **dadurch gekennzeichnet, dass** das Opioid oral verabreicht wird und das Devazepid oral verabreicht wird.

20. Monophasische pharmazeutische Zusammensetzung gemäß Anspruch 11, **dadurch gekennzeichnet, dass** das Opioid mittels intravenöser Verabreichung oder oraler Verabreichung verabreicht wird.

21. Monophasische pharmazeutische Zusammensetzung gemäß Anspruch 11, **dadurch gekennzeichnet, dass** das Opioid mittels transdermalem Pflaster verabreicht wird.

22. Monophasische pharmazeutische Zusammensetzung gemäß Anspruch 11, **dadurch gekennzeichnet, dass** für die orale Verabreichung die Tagesdosis von Devazepid von 0,07 mg/kg/Tag bis 0,7 mg/kg/Tag beträgt.

23. Monophasische pharmazeutische Zusammensetzung gemäß Anspruch 22, **dadurch gekennzeichnet, dass** für die orale Verabreichung die Tagesdosis von Devazepid von 0,07 mg/kg/Tag bis 0,29 mg/kg/Tag beträgt.

24. Monophasische pharmazeutische Zusammensetzung gemäß Anspruch 23, **dadurch gekennzeichnet, dass** für die intravenöse Verabreichung die Dosierung von Devazepid 50 µg/kg/Tag bis 0,5 mg/kg/Tag beträgt.

25. Monophasische pharmazeutische Zusammensetzung gemäß Anspruch 10, **dadurch gekennzeichnet, dass** das Opioid ausgewählt ist aus der Gruppe Morphin, oder einem seiner Salze, wie zum Beispiel dem Sulfat, Chlorid oder Hydrochlorid, oder den anderen 1,4-Hydroxymorphinan-Opioid-Analgetika, wie zum Beispiel Naloxon, Meperidin, Butorphanol oder Pentazocin, oder Morphin-6-glucuronid, Codein, Dihydrocodein, Diamorphin, Dextropropoxyphen, Pethidin, Fentanyl, Alfentanil, Alphaprodin, Buprenorphin, Dextromoramid, Diphenoxylat, Dipipanon, Heroin (Diacetylmorphin), Hydrocodon (Dihydrocodeinon), Hydromorphon (Dihydromorphinon), Levorphanol, Meptazinol, Methadon, Metopon (Methyldihydromorphinon), Nalbuphin, Oxycodon (Dihydrohydroxycodeinon), Oxymorphon (Dihydrohydroxymorphinon), Phenadoxon, Phenazocin, Remifentanil, Tramadol oder einem von deren Salzen, oder irgendeiner Kombination der oben genannten Verbindungen.

26. Monophasische pharmazeutische Zusammensetzung gemäß Anspruch 25, **dadurch gekennzeichnet, dass** das Opioid ausgewählt ist aus der der Gruppe Hydromorphon, Oxycodon, Morphin und Fentanyl oder einem von deren Salzen.

27. Monophasische pharmazeutische Zusammensetzung gemäß Anspruch 26, **dadurch gekennzeichnet, dass** das Opioid Morphin oder Morphinsulfat ist.

28. Monophasische pharmazeutische Zusammensetzung gemäß Anspruch 26, **dadurch gekennzeichnet, dass** das Opioid Fentanyl oder eines seiner Salze ist.

29. Monophasische pharmazeutische Zusammensetzung gemäß Anspruch 3, **dadurch gekennzeichnet, dass** die Tagesdosis von Oberflächenaktivstoff bis zu 0,056 mg/kg/Tag beträgt.

30. Monophasische pharmazeutische Zusammensetzung gemäß Anspruch 3, **dadurch gekennzeichnet, dass** die Tagesdosis von Oberflächenaktivstoff von 0,4 mg bis 1,6 mg pro Tag beträgt.

31. Monophasische pharmazeutische Zusammensetzung gemäß Anspruch 10, **dadurch gekennzeichnet, dass** die Dosierung eines Opioids von 5 bis 2000 mg täglich ist.

32. Monophasische pharmazeutische Zusammensetzung gemäß Anspruch 31, **dadurch gekennzeichnet, dass** die Dosierung des Opioids von 10 bis 240 mg täglich ist.

33. Monophasische pharmazeutische Zusammensetzung gemäß Anspruch 32, **dadurch gekennzeichnet, dass** die Tagesdosis des Opioids von 5 bis 100 mg täglich ist.

34. Monophasische pharmazeutische Zusammensetzung gemäß Anspruch 3, **dadurch gekennzeichnet, dass** das Devazepid im Wesentlichen das S-Enantiomer ist.

35. Monophasische pharmazeutische Zusammensetzung gemäß Anspruch 34, **dadurch gekennzeichnet, dass** der Anteil an R-Enantiomer, welches als eine Verunreinigung vorliegen kann, nicht größer als 1,5 % Gewicht/Gewicht ist.

36. Monophasische pharmazeutische Zusammensetzung gemäß Anspruch 3, **dadurch gekennzeichnet, dass** der Oberflächenaktivstoff ausgewählt ist aus der Gruppe Docusat-Natrium (Dioctylnatriumsulfosuccinat), Natriumdodecylsulfat und Tetradecyltrimethylammoniumbromid.

37. Monophasische pharmazeutische Zusammensetzung gemäß Anspruch 3, **dadurch gekennzeichnet, dass** der Oberflächenaktivstoff auch antimikrobielle und/oder antiseptische Eigenschaften besitzt.

38. Monophasische pharmazeutische Zusammensetzung gemäß Anspruch 37, **dadurch gekennzeichnet, dass** der Oberflächenaktivstoff Cetrimid ist.

39. Monophasische pharmazeutische Zusammensetzung gemäß Anspruch 36, **dadurch gekennzeichnet, dass** der Oberflächenaktivstoff Docusat-Natrium ist.

40. Monophasische pharmazeutische Zusammensetzung gemäß Anspruch 3, **dadurch gekennzeichnet, dass** die Zusammensetzung einen oder mehrere Füllstoffe umfasst.

41. Monophasische pharmazeutische Zusammensetzung gemäß Anspruch 40, **dadurch gekennzeichnet, dass** die Füllstoffteilchen mit dem Oberflächenaktivstoff beschichtet sind, wobei der beschichtete Füllstoff und Devazepid dann zu einer innigen Mischung vereinigt werden.

42. Monophasische pharmazeutische Zusammensetzung gemäß Anspruch 40, **dadurch gekennzeichnet, dass** der Füllstoff ausgewählt ist aus der Gruppe Lactose, Mannitol, Talkum, Magnesiumstearat, Natriumchlorid, Kaliumchlorid, Zitronensäure, sprühgetrocknete Lactose, hydrolysierte Stärken, Stärke, mikrokristalline Cellulose, Cellulosederivate, Sorbitol, Saccharose, Saccharose-basierte Materialien, Icodextrin, Calciumsulfat, Calciumhydrogenphosphat und Dextrose und deren Mischungen.

43. Monophasische pharmazeutische Zusammensetzung gemäß Anspruch 42, **dadurch gekennzeichnet, dass** der Füllstoff Stärke ist.

44. Monophasische pharmazeutische Zusammensetzung gemäß Anspruch 43, **dadurch gekennzeichnet, dass** die Stärke Maisstärke ist.

45. Monophasische pharmazeutische Zusammensetzung gemäß Anspruch 40, **dadurch gekennzeichnet, dass** die Größe der Devazepid-Teilchen und der Füllstoff-Teilchen unterschiedlich ist.

46. Monophasische pharmazeutische Zusammensetzung gemäß Anspruch 3, **dadurch gekennzeichnet, dass** das Verhältnis von Devazepid:Oberflächenaktivstoff von 5:1 bis 25:1 Gewicht/Gewicht ist.

47. Monophasische pharmazeutische Zusammensetzung gemäß Anspruch 40, **dadurch gekennzeichnet, dass** die Zusammensetzung Devazepid und einen Oberflächenaktivstoff umfasst, wobei der Rest der Zusammensetzung durch einen Füllstoff ausgeglichen ist.

48. Monophasische pharmazeutische Zusammensetzung gemäß Anspruch 47, **dadurch gekennzeichnet, dass** die Zusammensetzung 1,25 mg Devazepid, 0,1 mg Oberflächenaktivstoff und 148,65 mg eines Füllstoffs umfasst.

49. Monophasische pharmazeutische Zusammensetzung gemäß Anspruch 48, **dadurch gekennzeichnet, dass** die Zusammensetzung 1,25 mg Devazepid, 0,1 mg Docusat-Natrium und 148,65 mg Maisstärke umfasst.

50. Monophasische pharmazeutische Zusammensetzung gemäß Anspruch 47, **dadurch gekennzeichnet, dass** die Zusammensetzung 2,5 mg Devazepid, 0,2 mg Oberflächenaktivstoff und 297,3 mg eines Füllstoffs umfasst.

51. Monophasische pharmazeutische Zusammensetzung gemäß Anspruch 50, **dadurch gekennzeichnet, dass** die Zusammensetzung 2,5 mg Devazepid, 0,2 mg Docusat-Natrium und 297,3 mg Maisstärke umfasst.

52. Monophasische pharmazeutische Zusammensetzung gemäß Anspruch 3, **dadurch gekennzeichnet, dass** die Zusammensetzung in eine Kapsel gefüllt ist.

53. Monophasische pharmazeutische Zusammensetzung gemäß Anspruch 52, **dadurch gekennzeichnet, dass** die Kapsel eine Gelatinekapsel ist.

## Revendications

1. Utilisation de devazepide dans la fabrication d'une composition pharmaceutique monophasique comportant une quantité thérapeutiquement efficace de devazepide et un surfactant pharmaceutiquement acceptable où le dosage quotidien du devazepide est jusqu'à 0,7 mg/kg/jour, **caractérisée en ce que** le surfactant est choisi dans le groupe des sulfosuccinates d'alkyl, des sulfates d'alkyl ou des sels d'ammonium d'alkyl.

2. Utilisation selon la revendication 1, **caractérisée en ce que** la composition pharmaceutique est sous la forme d'un dosage solide.

3. Composition pharmaceutique monophasique comportant une quantité thérapeutiquement efficace de devazepide et un surfactant pharmaceutiquement acceptable où le dosage quotidien du devazepide est jusqu'à 0,7 mg/kg/jour, **caractérisée en ce que** le surfactant est choisi dans le groupe des sulfosuccinates d'alkyl, des sulfates d'alkyl ou des sels d'ammonium d'alkyl.

4. Composition pharmaceutique monophasique selon la revendication 3, **caractérisée en ce que** le dosage quotidien du devazepide est de 25 µg/kg/jour à 0,7 mg/kg/jour.

5. Composition pharmaceutique monophasique selon la revendication 4, **caractérisée en ce que** le dosage quotidien du devazepide est de 50 µg /kg/jour à 0,5 mg/kg/jour.

6. Composition pharmaceutique monophasique selon la revendication 3, **caractérisée en ce que** la composition est sous une forme liquide.

7. Composition pharmaceutique monophasique selon la revendication 3, **caractérisée en ce que** la composition est sous une forme d'un dosage solide.

8. Composition pharmaceutique monophasique selon la revendication 7, **caractérisée en ce que** la composition est sous forme d'un comprimé.

9. Composition pharmaceutique monophasique selon la revendication 7, **caractérisée en ce que** la composition est sous forme de poudre fluide dans une capsule.

10. Composition pharmaceutique monophasique selon la revendication 3, **caractérisée en ce que** la composition est adaptée pour l'administration séparée, simultanée ou séquentielle avec une quantité thérapeutiquement efficace d'un analgésique d'opioïde.

11. Composition pharmaceutique monophasique selon la revendication 3, **caractérisée en ce que** la composition est adaptée pour être administrée en intraveineuse, de manière intra artérielle, orale, intrathécale, intra nasale, intra rectale, intramusculaire /sous-cutanée, par inhalation ou par un patch transdermique.

12. Composition pharmaceutique monophasique selon la revendication 11, **caractérisée en ce que** le devazepide et/ou l'opioïde est adapté pour être administrée en intraveineuse.

13. Composition pharmaceutique monophasique selon la revendication 12, **caractérisée en ce que** l'administration intraveineuse est réalisée par un bol intraveineux ou une infusion intraveineuse continue.

14. Composition pharmaceutique monophasique selon la revendication 11, **caractérisée en ce que** le devazepide et/ou l'opioïde est adaptée pour être administrée par voie sous-cutanée.

15. Composition pharmaceutique monophasique selon la revendication 14, **caractérisée en ce que** l'administration sous-cutanée est du type infusion sous-cutanée.

16. Composition pharmaceutique monophasique selon la revendication 11, **caractérisée en ce que** le devazepide et/ou l'opioïde est adaptée pour être administrée oralement.

17. Composition pharmaceutique monophasique selon la revendication 11, **caractérisée en ce que** le devazepide est administrée oralement.

18. Composition pharmaceutique monophasique selon la revendication 12, **caractérisée en ce que** l'opioïde est administré en intraveineuse et le devazepide est administré en intraveineuse.

19. Composition pharmaceutique monophasique selon la revendication 16, **caractérisée en ce que** l'opioïde est administrée oralement et le devazepide est administré oralement.

20. Composition pharmaceutique monophasique selon la revendication 11, **caractérisée en ce que** l'opioïde est administrée par administration intraveineuse ou administration orale.

21. Composition pharmaceutique monophasique selon la revendication 11, **caractérisée en ce que** l'opioïde est administrée par un patch transdermique.

22. Composition pharmaceutique monophasique selon la revendication 11, **caractérisée en ce que**, pour l'administration par voie orale, le dosage quotidien du devazepide est de 0,07 mg/kg/jour à 0,7 mg/kg/jour.

23. Composition pharmaceutique monophasique selon la revendication 22, **caractérisée en ce que**, pour l'administration par voie orale, le dosage quotidien du devazepide est de 0,07 mg/kg/jour à 0,29 mg/kg/jour.

24. Composition pharmaceutique monophasique selon la revendication 23, **caractérisée en ce que**, pour l'administration intraveineuse, le dosage du devazepide est 50 µg /kg/jour à 0,5 mg/kg/jour.

25. Composition pharmaceutique monophasique selon la revendication 10, **caractérisée en ce que** l'opioïde est choisi parmi la morphine ou un sel de celui-ci tel que le sulfate, le chlorure ou le chlorhydrate, ou les autres analgésiques de l'opioïde 1,4-hydroxymorphinane tels que le naloxone, le meperidine, le butorphanol ou le pentazocine, ou le morphine-6-glucuronide, la codéine, dihydrocodéine, diamorphine, dextropropoxyphene, pethidine, fentanyle, alfentanil, alphaprodine, buprenorphine, dextromoramide, diphenoxylate, dipipanone, héroïne (diacetylmorphine), hydrocodone (dihydrocodeinone), hydromorphone (dihydromorphinone), levorphanol, meptazinol, méthadone, metopone (methyldihydromorphinone), nalbuphine, oxycodone (dihydrohydroxycodeinone), oxymorphone (dihydrohydroxymorphinone), phenadoxone, phenazocine, le remifentanil, tramadol, ou un sel de ces derniers, ou de toute combinaison des composés mentionnés ci-dessus.

26. Composition pharmaceutique monophasique selon la revendication 25, **caractérisée en ce que** l'opioïde est choisi dans le groupe de l'hydromorphone, l'oxycodone, la morphine et le fentanyle, ou un sel de ceux-ci.

27. Composition pharmaceutique monophasique selon la revendication 26, **caractérisée en ce que** l'opioïde est la morphine ou la sulfate de morphine.

28. Composition pharmaceutique monophasique selon la revendication 26, **caractérisée en ce que** l'opioïde est le fentanyl, ou un sel de celui-ci.

29. Composition pharmaceutique monophasique selon la revendication 3, **caractérisée en ce que** la dose quotidienne de surfactant est jusqu'à 0,056 mg/kg/jour.

30. Composition pharmaceutique monophasique selon la revendication 3, **caractérisée en ce que** la dose quotidienne de surfactant est de 0.4mg à 1.6mg par jour.

31. Composition pharmaceutique monophasique selon la revendication 10, **caractérisée en ce que** le dosage d'un opioïde est de 5 à 2000mg par jour.

32. Composition pharmaceutique monophasique selon la revendication 31, **caractérisée en ce que** le dosage de l'opioïde est de 10 à 240mg par jour.

33. Composition pharmaceutique monophasique selon la revendication 32, **caractérisée en ce que** le dosage quotidien de l'opioïde est de 5 à 100mg par jour.

34. Composition pharmaceutique monophasique selon la revendication 3, **caractérisée en ce que** le devazepide est sensiblement l'énantiomère S.

35. Composition pharmaceutique monophasique selon la revendication 34, **caractérisée en ce que** le niveau de l'énantiomère R, qui peut être présent comme impureté, n'est pas supérieur à 1,5% poids/poids.

36. Composition pharmaceutique monophasique selon la revendication 3 **caractérisée en ce que** le surfactant est choisi parmi le groupe de : le sodium de docusate (sulfosuccinate dioctyl de sodium), le sulfate dodécyl de sodium et le bromure d'ammonium de tetradecyltrimethyl.

37. Composition pharmaceutique monophasique selon la revendication 3, **caractérisée en ce que** le surfactant possède également des propriétés antimicrobiennes et/ou antiseptiques.

38. Composition pharmaceutique monophasique selon la revendication 37, **caractérisée en ce que** le surfactant est la cetrimide.

39. Composition pharmaceutique monophasique selon la revendication 36, **caractérisée en ce que** le surfactant est le sodium de docusate.

40. Composition pharmaceutique monophasique selon la revendication 3, **caractérisée en ce que** la composition comporte un ou plusieurs remplisseurs.

41. Composition pharmaceutique monophasique selon la revendication 40, **caractérisée en ce que** les particules de remplisseur sont revêtues avec du surfactant, le remplisseur revêtu et le devazepide étant alors formés dans un mélange intime.

42. Composition pharmaceutique monophasique selon la revendication 40, **caractérisée en ce que** le remplisseur est choisi parmi le lactose, le mannitol, le talc, le stéarate de magnésium, le chlorure de sodium, le chlorure de potassium, l'acide citrique, le lactose séché au vaporisateur, les amidons hydrolysés, l'amidon, la cellulose microcristalline, les cellulosiques, le sorbitol, le sucrose, les matériaux à base de sucrose, l'icodextrine, le sulfate de calcium, le phosphate de calcium et le dextrose dibasique et leurs mélanges.

43. Composition pharmaceutique monophasique selon la revendication 42, **caractérisée en ce que** le remplisseur est l'amidon.

44. Composition pharmaceutique monophasique selon la revendication 43, **caractérisée en ce que** l'amidon est du fécule de maïs.

45. Composition pharmaceutique monophasique selon la revendication 40, **caractérisée en ce que** la taille des particules de devazepide et des particules de remplisseur sont différentes.

46. Composition pharmaceutique monophasique selon la revendication 3, **caractérisée en ce que** le rapport devazepide: surfactant est de 5: 1 à 25: 1 poids/poids.

47. Composition pharmaceutique monophasique selon la revendication 40, **caractérisée en ce que** la composition comporte du devazepide et un surfactant avec le reste de la composition avec un remplisseur.

48. Composition pharmaceutique monophasique selon la revendication 47, **caractérisée en ce que** la composition comporte 1.25 mg de devazepide, 0,1 mg de surfactant et 148,65 mg d'un remplisseur.

49. Composition pharmaceutique monophasique selon la revendication 48, **caractérisée en ce que** la composition comporte 1.25mg de devazepide, 0,1 mg de sodium de docusate et 148,65 mg de fécule de maïs.

50. Composition pharmaceutique monophasique selon la revendication 47, **caractérisée en ce que** la composition comporte 2.5 mg de devazepide, 0,2 mg de surfactant et 297.3mg d'un remplisseur.

51. Composition pharmaceutique monophasique selon la revendication 50, **caractérisée en ce que** la composition comporte 2.5 mg de devazepide, 0.2 mg de sodium de docusate et 297.3 mg de fécule de maïs.

52. Composition pharmaceutique monophasique selon la revendication 3, **caractérisée en ce que** la composition est versée dans une capsule.

53. Composition pharmaceutique monophasique selon la revendication 52, **caractérisée en ce que** la capsule est une capsule de gélatine.
